# EUROPEAN PATENT APPLICATION

(11) **EP 1 990 332 A1**
(43) Date of publication of application: **12.11.2008**
(21) Application number: 07714792.4
(22) Date of filing: 22.02.2007
(51) Int. Cl.: C07C 15/38, C09K 11/06, H01L 51/50

(54) **NAPHTHACENE DERIVATIVE AND ORGANIC ELECTROLUMINESCENT DEVICE USING SAME**

(30) Priority: 28.02.2006 JP 2006053018
(71) Applicant: Idemitsu Kosan Co., Ltd., Chiyoda-ku Tokyo 100-8321 (JP)
(72) Inventor: ARAKANE, Takashi, Sodegaura-shi, Chiba 2990293 (JP); IKEDA, Kiyoshi, Sodegaura-shi, Chiba 2990293 (JP); YAMAMOTO, Hiroshi, Sodegaura-shi, Chiba 2990293 (JP); SADO, Takayasu, Sodegaura-shi, Chiba 2990293 (JP); HOSOKAWA, Chishio, Sodegaura-shi, Chiba 2990293 (JP)
(74) Representative: Gille Hrabal Struck Neidlein Prop Roos
(86) International application number: PCT/JP2007/053294
(87) International publication number: WO 2007/105448

(57) **Abstract**

A material for an organic EL device conatining a novel naphthacene derivative; a luminescent material conatining a novel naphthacene derivative; and an organic electroluminescence device including one or more organic layers interposed between a cathode and an anode, and at least one layer of the organic layers containing a naphthacene derivative represented by the following formula (1) or (2).

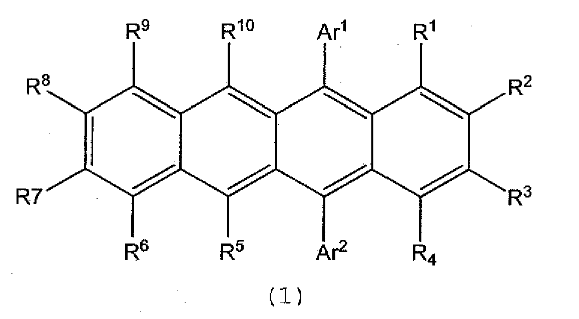

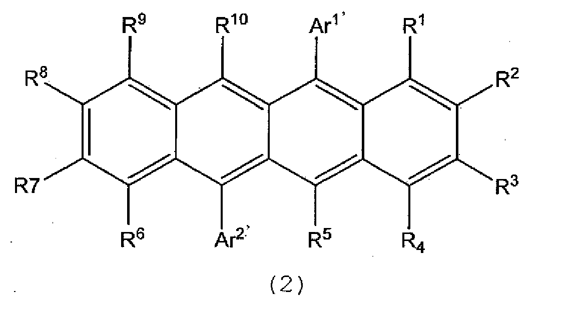

## Description

### TECHNICAL FIELD

The invention relations to a novel naphthacene derivative, a material for an organic EL device using the same and an organic EL device containing the same.

### BACKGROUND

An organic EL device is a self-emission device by the use of the principle that a fluorescent material emits light by the recombination energy of holes injected from an anode and electrons injected from a cathode when an electric field is impressed.

Since C. W. Tang et al. of Eastman Kodak Co. reported a low-voltage driven organic EL device of stack type (Non-patent Document 1), studies on organic EL devices wherein organic materials are used as constituent materials has actively conducted.

Tang et al. uses tris(8-quinolinol) aluminum (Alq) for an emitting layer and a triphenyldiamine derivative for a hole-transporting layer in a stack structure. The advantages of the stack structure are to increase injection efficiency of holes to the emitting layer, to increase generation efficiency of excitons generated by recombination by blocking electrons injected in the cathode, to confine the generated excitons in the emitting layer, and so on. Like this example, as the structure of the organic EL device, a two-layered type of a hole-transporting (injecting) layer and an electron-transporting emitting layer, and a three-layered type of a hole-transporting (injecting) layer, an emitting layer and an electron-transporting (injecting) layer are widely known. In such stack structure devices, their device structures and fabrication methods have been contrived to increase recombination efficiency of injected holes and electrons.

As a luminescent material used in an organic EL device, there are known chelate complexes such as tris(8-quinolinol) aluminum complexes, coumarin complexes, tetrapenylbutadiene derivatives, bisstyrlarylene derivatives, oxadiazole derivatives and the like. They are reported to give emission in the visible range from blue to red, and are expected to realize color display devices (Patent documents 1 to 3, for example). However they do not have a practical luminous efficiency and lifetime. Further, full-color displays require three primary colors (blue, green, red), particularly a high efficient red device.

For example, Patent document 4 has recently disclosed a red luminescent device where a naphthacene or pentacene derivative is added in an emitting layer. This luminescent device is excellent in red purity but its applied voltage is high of 11 V and the halftime of luminance is insufficient of about 150 hours. Patent document 5 discloses a device where a dicyanomethylene (DCM) compound is added in an emitting layer but the red purity thereof is not satisfactory. Patent document 6 discloses a red luminescent device where an amine type aromatic compound is added in an emitting layer. This device has a good color purity of CIE chromaticity (0.64, 0.33), but the driven voltage is high. Patent documents 7 and 8 disclose devices using an amine type aromatic compound and Alq in an emitting layers The device emits red light with low efficiency and short lifetime.

Patent document 9 discloses a device using an amine type aromatic compound and DPVDPAN in an emitting layer. The device emits orange light at a high efficiency but red light at a low efficiency.

Patent document 10 discloses a device where a dicyanoanthracene derivative and an indenoperylene derivative are used in an emitting layer, and a metal complex is used in an electron-transporting layer. However, the emission color thereof is reddish orange.

Patent document 11 discloses a device wherein a naphthacene derivative and an indenoperylene derivative are used in an emitting layer, and a naphthacene derivative is used in an electron-transporting layer; however, it does not have a practical efficiency.

[Patent document 1] JP-A-H8-239655
[Patent document 2] JP-A-H7-138561
[Patent document 3] JP-A-H3-200289
[Patent document 4] JP-A-H8-311442
[Patent document 5] JP-A-H3-162481
[Patent document 6] JP-A-2001-81451
[Patent document 7] WO01/23497
[Patent document 8] JP-A-2003-40845
[Patent document 9] JP-A-2003-81924
[Patent document 10] JP-A-2001-307885
[Patent document 11] JP-A-2003-338377
[Non-patent document 1] C.W. Tang, S.A. Vanslyke, Applied Physics Letters, 51, 913, 1987

An object of the invention is to solve the above-mentioned subject, and to provide a novel naphthacene derivative useful as a material of an organic EL device and an organic EL device with a practical efficiency and lifetime using the naphthacene derivative in at least one layer of organic compound layers.

### Disclosure of the Invention

The inventors made extensive studies to attain the above object, and have found that the lifetime and efficiency of an organic EL device can be enhanced by using a novel naphthacene derivative with a specific structure in at least one layer of organic compound layers. The invention has made based on the finding. The invention provides the following naphthacene derivative, material or luminescent material for an organic EL device containing the naphthacene derivative, organic EL device, and apparatus therewith.

1. A naphthacene derivative represented by the following formula (1) or (2): 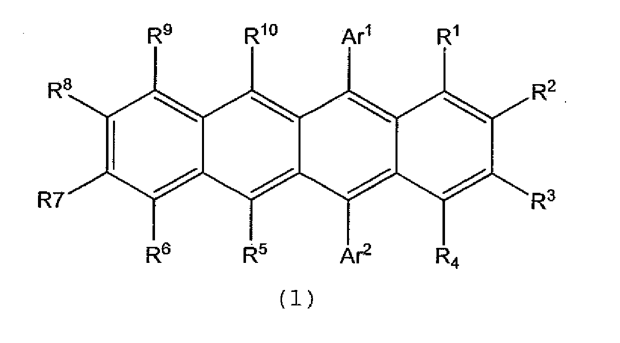 wherein Ar¹ and Ar² are not the same as each other, and are a substituted or unsubstituted aromatic group having 6 to 50 of carbon atoms that form a ring (ring carbon atoms); and R¹ to R¹⁰ are each independently a hydrogen atom, substituted or unsubstituted aromatic group having 6 to 50 ring carbon atoms, or substituted or unsubstituted alkyl group having 1 to 50 carbon atoms; 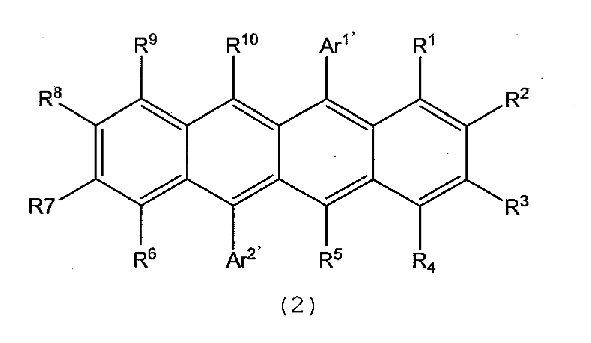 wherein Ar^{1'} and Ar^{2'} may be the same as each other, and are a substituted or unsubstituted aromatic group having 6 to 50 ring carbon atoms; and R¹ to R¹⁰ are each independently a hydrogen atom, substituted or unsubstituted aromatic group having 6 to 50 ring carbon atoms, or substituted or unsubstituted alkyl group having 1 to 50 carbon atoms.
2. The naphthacene derivative according to 1 which is selected from the group consisting of compounds represented by the following formulas (3) and (4): 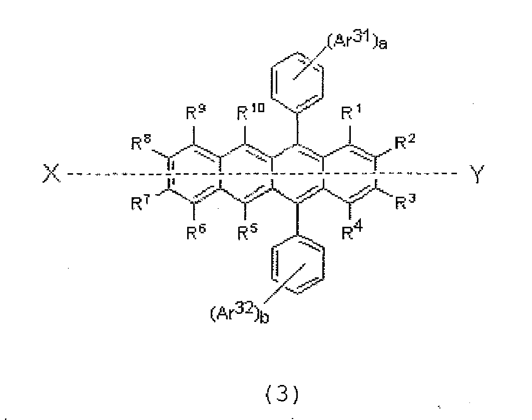 wherein Ar³¹ and Ar³² are each independently a substituted or unsubstituted aromatic group having 6 to 50 ring carbon atoms; R¹ to R¹⁰ are each independently a hydrogen atom, substituted or unsubstituted aromatic group having 6 to 50 ring carbon atoms, or substituted or unsubstituted alkyl group having 1 to 50 carbon atoms; and a and b are each an integar of 0 to 5, provided that groups do not symmetrically bond to 5 and 12 positions of the central naphthacene with respect to the X-Y axis; 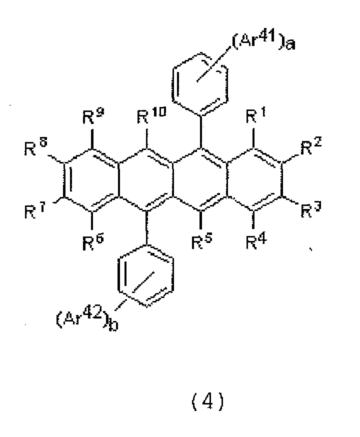 wherein Ar⁴¹ and Ar⁴² are each independently a substituted or unsubstituted aromatic group having 6 to 50 ring carbon atoms; R¹ to R¹⁰ are each independently a hydrogen atom, substituted or unsubstituted aromatic group having 6 to 50 ring carbon atoms, or substituted or unsubstituted alkyl group having 1 to 50 carbon atoms; and a and b are each an integer of 0 to 5.
3. A material for an organic electroluminescence device comprising the naphthacene derivative of 1 or 2.
4. A luminescent material for an organic electroluminescence device comprising the naphthacene derivative of 1 or 2.
5. An organic electroluminescence device comprising:
   at least one organic layer interposed between a cathode and an anode, and
   the organic layer containing the naphthacene derivative of 1 or 2.
6. The organic electroluminescence device according to 5 wherein the organic layer containing the naphthacene derivative of 1 or 2 is an emitting layer.
7. An organic electroluminescence device comprising:
   an emitting layer and an electron-transporting layer between a cathode and an anode, and
   the emitting layer containing a host material that is the naphthacene derivative of 1 or 2 and a dopant material that is an indenoperylene derivative.
8. The organic electroluminescence device according to 7 wherein the electron-transporting layer contains a compound represented by the following formula (5),

   A-B (5)

   wherein A is an aromatic hydrocarbon group with three or more carbocircles and B is a substituted or unsubstituted heterocyclic group.
9. The organic electroluminescence device according to 8 wherein the compound represented by the formula (5) is a compound containing in the molecule thereof at least one skeleton selected from anthracene, phenanthrene, naphthacene, pyrene, chrysene, benzoanthracene, pentacene, dibenzoanthracene, benzopyrene, fluorene, benzofluorene, fluoranthene, benzofluoranthene, naphthofluoranthene, dibenzofluorene, dibenzopyrene and dibenzofluoranthene.
10. The organic electroluminescence device according to 8 wherein the compound represented by the formula (5) is a nitrogen-containing heterocyclic compound.
11. The organic electroluminescence device according to 10 wherein the nitrogen-containing heterocyclic compound is a nitrogen-containing heterocyclic compound containing in the molecule thereof at least one skeleton selected from pyridine, pyrimidine, pyrazine, pyridazine, triazine, quinoline, quinoxaline, acridine, imidazopyridine, imidazopyrimidine and phenenthroline.
12. The organic electroluminescence device according to 10 wherein the nitrogen-containing heterocyclic compound is a benzoimidazole derivative represented by formula (6) or (7), 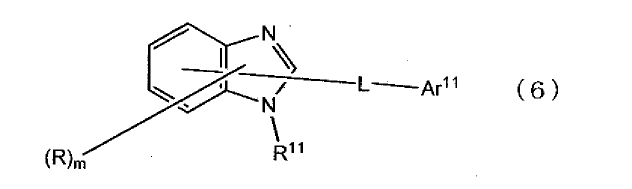 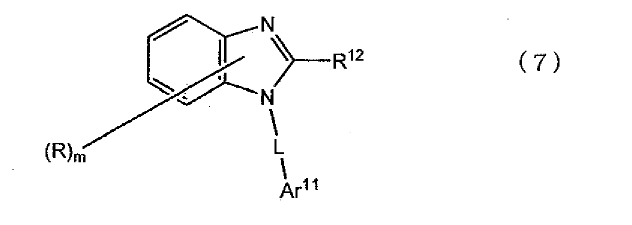 wherein R is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or substituted or unsubstituted alkoxy group having 1 to 20 carbon atoms;
   m is an integer of 0 to 4;
   R¹¹ is a substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or alkoxy group having 1 to 20 carbon atoms;
   R¹² is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or substiuted or unsubstituted alkoxy group having 1 to 20 carbon atoms;
   L is a substituted or unsubstituted arylene group having 6 to 60 carbon atoms, substituted or unsubstituted pyridinylene group, substituted or unsubstituted quinolinylene group, or substituted or unsubstituted fluorenylene group; and
   Ar¹¹ is a substituted or unsubstituted aryl group having 6 to 60 carbon atoms, substituted or unsubstituted pyridinyl group, or substituted or unsubstituted quinolinyl group.
13. The organic electroluminescence device according to any one of 7 to 12 wherein the indenoperylene derivative of the dopant material in the emitting layer is one or more compounds selected from the group consisting of indenoperylene derivatives represented by the following formulas (12) and (13): 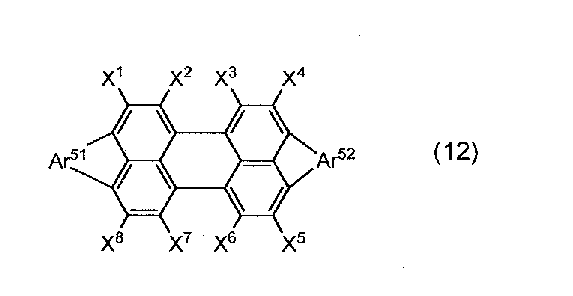 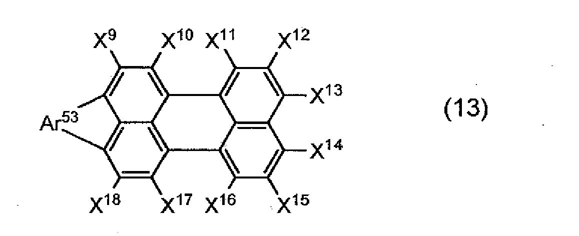 wherein Ar⁵¹, Ar⁵² and Ar⁵³ are each independently a substituted or unsubstiuted aromatic hydrocarbon group having 6 to 50 ring carbon atoms, or substituted or unsubstituted aromatic heterocyclic group having 6 to 50 of atoms that form a ring (ring atoms); X¹ to X¹⁸ are each independently a hydrogen atom, halogen atom, substituted or unsubstituted alkyl group having 1 to 50 carbon atoms, substituted or unsubstituted alkoxy group having 1 to 50 carbon atoms, substituted or unsubstituted alkylthio group having 1 to 50 carbon atoms, substituted or unsubstituted alkenyl group having 2 to 50 carbon atoms, substituted or unsubstiuted alkenyloxy group having 1 to 50 carbon atoms, substituted or unsubstituted alkenylthio group having 1 to 50 carbon atoms, substiuted or unsubstituted aromatic hydrocarbon group having 6 to 50 ring carbon atoms, substiuted or unsubstiuted aromatic heterocyclic group having 6 to 50 ring atoms, substiuted or unsubstiuted aryloxy group having 6 to 50 ring carbon atoms, substituted or unsubstiuted arylthio group having 6 to 50 ring carbon atoms, substiuted or unsubstiuted aralkyl group having 7 to 50 ring carbon atoms, substiuted or unsubstituted arylalkyloxy group having 6 to 50 ring carbon atoms, substiuted or unsubstiuted arylalkylthio group having 6 to 50 ring carbon atoms, substiuted or unsubstiuted arylalkenyl group having 6 to 50 ring carbon atoms, substiuted or unsubstiuted alkenylaryl group having 6 to 50 ring carbon atoms, amino group, carbazolyl group, cyano group, hydroxy group, -COOR⁵¹, -COR⁵² or -OCOR⁵³ (R⁵¹, R⁵² and R⁵³ are each a hydrogen atom, substituted or unsubstituted alkyl group having 1 to 50 carbon atoms, substituted or unsubstituted alkenyl group having 2 to 50 carbon atoms, substituted or unsubstituted aralkyl group having 7 to 50 ring carbon atoms, substituted or unsubstituted aromatic hydrocarbon group having 6 to 50 ring carbon atoms, or substituted or unsubstituted aromatic heterocyclic group having 6 to 50 ring atoms); adjacent groups of X¹ to X¹⁸ may be bonded to each other to form a ring with a substituted carbon atom; and at least one of X¹ to X¹⁸ is not a hydrogen atom.
14. The organic electroluminescence device according to any one of 7 to 13 wherein the indenoperylene derivative of the dopant material in the emitting layer is a dibenzo tetraphenyl periflanthene derivative.
15. The organic electroluminescence device according to any one of 7 to 14 wherein the doping concentration of the dopant in the emitting layer is 0.1 to 10 wt%.
16. The organic electroluminescence device according to 15 wherein the doping concentration of the dopant in the emitting layer is 0.5 to 2 wt%.
17. The organic electroluminescence device according to any one of 7 to 15 whose emission color is orange to red.
18. An apparatus comprising the organic electroluminescet device of any one of 7 to 17.

According to the invention, an organic EL device with a high efficiency and long lifetime that is excellent in color purity can be provided.
According to the invention, an organic EL device with an even higher efficiency can be obtained by selecting compounds suitable for an electron-transporting layer and emitting layer. That is, an organic EL device with high color purity can be obtained where exciton generation in an electron-transporting layer is suppressed and therefore slight emission from the electron-transporting layer is further suppressed. The lifetime of the device can be extended for the same reasons.

### Brief Description of Drawing

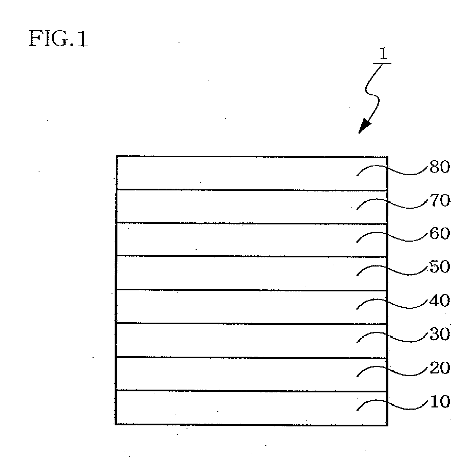

FIG. 1 is a view showing an embodiment according to the organic EL device of the invention.

### Best Mode for Carrying out the Invention

### I-1. Naphthacene derivative

The naphthacene derivative of the invention is represented in the following general formula (1) or (2).

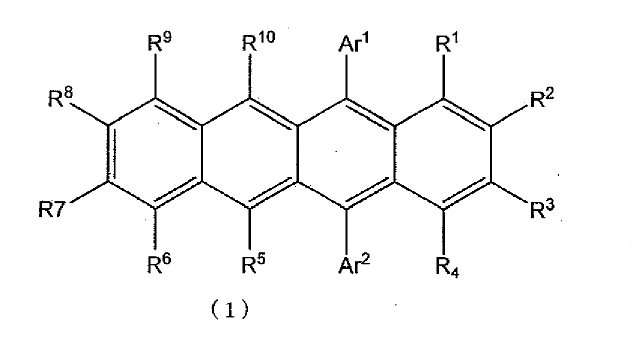

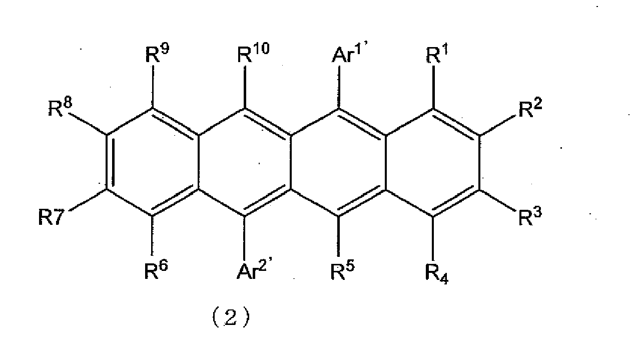

wherein Ar¹ and Ar² are not the same as each other, Ar^{1'} and Ar^{2'} may be the same as each other, and Ar¹, Ar², Ar^{1'} and Ar^{2'} are a substituted or unsubstituted aromatic group having 6 to 50 ring carbon atoms.
The unsubstituted aromatic group having 6 to 50 ring carbon atoms of Ar¹, Ar², Ar^{1'} and Ar^{2'} is preferably a phenyl group, (o-, m-, and p-) tolyl group, pyrenyl group, perylenyl group, coronenyl group, (1-, and 2-) naphthyl group, anthryl group, (o-, m-, and p-) biphenylyl group, terphenyl group, phenanthryl group or the like, and more preferably a phenyl group, (1-, and 2-) naphthyl group, (o-, m-, and p-) biphenylyl group, terphenyl group or the like.
A preferable combination of Ar¹ and Ar² when Ar¹ and Ar² are not the same as each other includes a combination in which Ar¹ is a phenyl group and Ar² is not a phenyl group.
When Ar^{1'} and Ar^{2'} are the same as each other, Ar^{1'} and Ar^{2'} are preferably a phenyl group, (o-, m-, and p-) tolyl group, pyrenyl group, perylenyl group, coronenyl group, (1-, and 2-) naphthyl group, anthryl group, (o-, m-, and p-) biphenylyl group, terphenyl group, phenanthryl group or the like, and more preferably a phenyl group, (1-, and 2-) naphthyl group, (o-, m-, and p-) biphenylyl group, terphenyl group or the like.
When Ar^{1'} and Ar^{2'} are not the same as each other, a preferable combination of Ar^{1'} and Ar^{2'} is the same as the above-mentioned combination of Ar¹ and Ar² when Ar¹ and Ar² are not the same as each other.

R¹ to R¹⁰ are each independently a hydrogen atom, substituted or unsubstituted aromatic group having 6 to 50 ring carbon atoms, or substituted or unsubstituted alkyl group having 1 to 50 carbon atoms.

Preferable unsubstituted aromatic group having 6 to 50 ring carbon atoms of R¹ to R¹⁰ includes a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylprapyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, fluoranthenyl group and fluorenyl group. More preferable are a phenyl group, 1-naphthyl group, 2-naphthyl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group, p-t-butylphenyl group or the like.

When R¹ to R¹⁰ are a substituted aromatic group having 6 to 50 ring carbon atoms, the substituent of the aromatic group includes a substituted or unsubstituted aryl group having 6 to 50 ring carbon atoms, substituted or unsubstituted aromatic heterocyclic group having 5 to 50 ring atoms, substituted or unsubstituted alkyl group having 1 to 50 carbon atoms, substituted or unsubstituted alkoxy group having 1 to 50 carbon atoms, substituted or unsubstituted aralkyl group having 1 to 50 carbon atoms, substituted or unsubstituted aryloxy group having 5 to 50 ring atoms, substituted or unsubstituted arylthio group having 5 to 50 ring atoms, substituted or unsubstituted carboxyl group having 1 to 50 carbon atoms, halogen group, cyano group, nitro group, hydroxy group and the like.

As examples of the substituted or unsubstituted aryl group having 6 to 50 ring carbon atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, fluoranthenyl group, and the like can be given.

As examples of the substituted or unsubstituted aromatic heterocyclic group having 5 to 50 ring atoms, a 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-5-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 10-phenothiadinyl group, 1-phenoxadinyl group, 2-phenoxadinyl group, 3-phenoxadinyl group, 4-phenoxadinyl group, 10-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butyl-pyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, and the like can be given.

As examples of the substituted or unsubstituted alkyl group, a methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group, 2-norbornyl group, and the like can be given.

The substituted or unsubstituted alkoxy group is a group shown by -OY. As examples of Y, a methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, and the like can be given.

Examples of the aralkyl group include benzyl, 1-phenylethyl, 2-phenylethyl, 1-phenylisopropyl, 2-phenylisopropyl, phenyl-t-butyl, α-naphthylmethyl, 1-α-naphthylethyl, 2-α-naphthylethyl, 1-α-naphthylisopropyl, 2-α-naphthylisopropyl, β-naphthylmethyl, 1-β-naphthylethyl, 2-β-naphthylethyl, 1-β-naphthylisopropyl, 2-β-naphthylisopropyl, 1-pyrrolylmethyl, 2-(1-pyrrolyl)ethyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl, and 1-chloro-2-phenylisopropyl groups.

The substituted or unsubstituted aryloxy group is shown by -OY'. As examples of Y', a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phen.anthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolzn-10-y1 group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, and the like can be given.

The substituted or unsubstituted arylthio group is shown by -SY''. As examples of Y'', a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthxolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolzn-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, and the like can be given.

The substituted or unsubstituted alkoxycarbonyl group is a group shown by -COOZ. As examples of Z, a methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, and the like can be given.

The divalent group forming a ring includes a tetramethylene group, pentamethylene group, hexamethylene group, diphenylmethane-2,2'-diyl group, diphenylethane-3,3'-diyl group and diphenylpropane-4,4'-diyl group.
As the halogen atom, fluorine, chlorine, bromine and iodine can be given.

The unsubstituted alkyl group having 1 to 50 carbon atoms of R¹ to R¹⁰ is preferably a methyl group, ethyl group, (n, i)-propyl group, (n, i, sec, tert)-butyl group, (n, i, neo, tert)-pentyl group or the like, and more preferably a methyl group or the like.

When R¹ to R¹⁰ are a substituted alkyl group having 1 to 50 carbon atoms, the substituent of the alkyl group includes a substituted or unsubstituted aryl group having 6 to 50 ring carbon atoms, substituted or unsubstituted aromatic heterocyclic group having 5 to 50 ring atoms, substituted or unsubstituted alkyl group having 1 to 50 carbon atoms, substituted or unsubstituted alkoxy group having 1 to 50 carbon atoms, substituted or unsubstituted aralkyl group having 1 to 50 carbon atoms, substituted or unsubstituted aryloxy group having 5 to 50 ring atoms, substituted or unsubstituted arylthio group having 5 to 50 ring atoms, substituted or unsubstituted carboxyl group having 1 to 50 carbon atoms, halogen group, cyano group, nitro group, hydroxy group and the like.

As preferable examples of the naphthacene derivative represented by the above-mentioned general formulas (1) and (2), the compounds represented by the following general formulas (3) and (4) can be given.

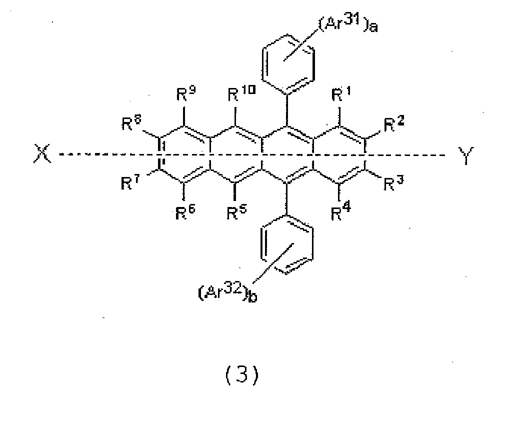

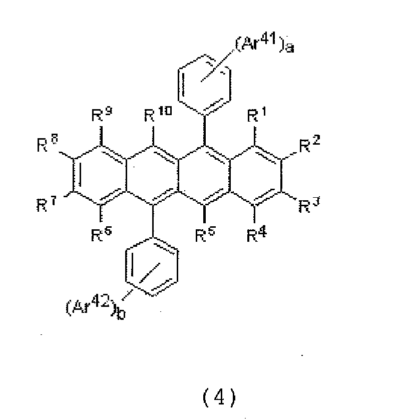

In the formula (3), Ar³¹ and Ar³² are each a substituted or unsubstituted aromatic group with 6 to 50 ring carbon atoms; R¹ to R¹⁰ are each a hydrogen atom, a substituted or unsubstituted aromatic group with 6 to 50 ring carbon atoms or a substituted or unsubstituted alkyl group with 1 to 50 carbon atoms; and a and b are each an integer of 0 to 5, provided that groups do not symmetrically bond to 5 and 12 positions of the central naphthacene with respect to X-Y axis.

In the formula (4), Ar⁴¹ and Ar⁴² are each a substituted or unsubstituted aromatic group with 6 to 50 ring carbon atoms; R¹ to R¹⁰ are each a hydrogen atom, a substituted or unsubstituted aromatic group with 6 to 50 ring carbon atoms or a substituted or unsubstituted alkyl group with 1 to 50 carbon atoms; and a and b are each an integer of 0 to 5.

The examples of substituents in formulas (3) and (4) are the same as those in formulas (1) and (2).

The naphthacene derivative of the invention represented by the general formulas (1) and (2) can be synthesized by using 5,12-naphthacene quinone or 5,11-naphthacenequinone as a start material. As a synthesis method for 5,11-naphthacene quinone, a method in which naphthacene is bromonated and oxidized with sulfuric acid (Bull. Chim. Soc. Fr., (1948) p.418-428), and a method in which dibenzylidenesuccinic acid is cyclized with sulfuric acid (Compt Rendus Seances Acad Sci., 206, (1938) p.756-759) can be used.

A typical synthesis scheme is shown below.

### <Naphthacene of general formula (1) >

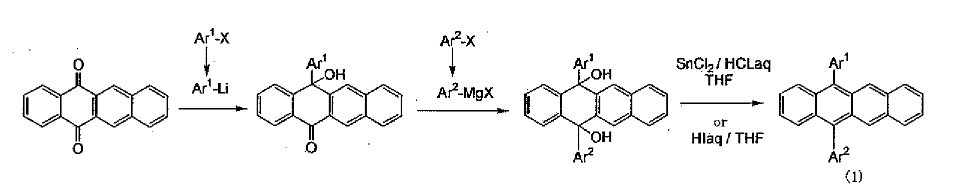

### <Naphthacene of general formula (2) >

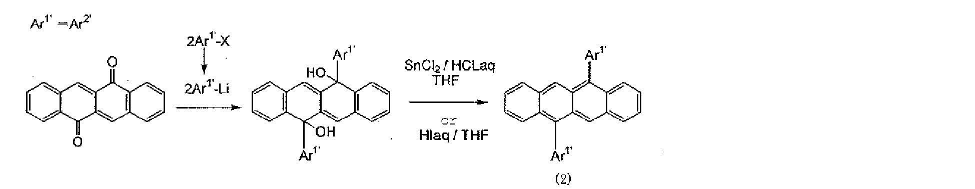

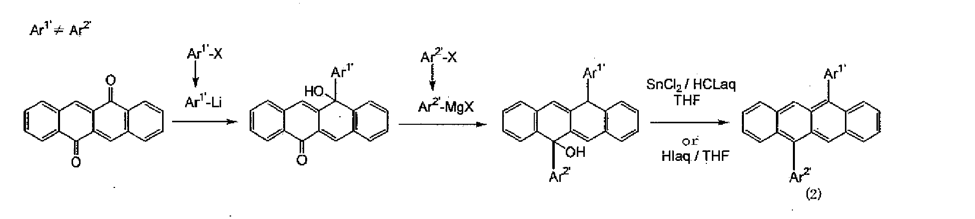

### I-2. Material for organic EL device including naphthacene derivative

The material for an organic EL device of the invention is characterized by containing the above naphthacene derivative of the invention.

The use of the naphthacene derivative of the invention in at least one layer of organic compound layers of an organic EL device can realize an organic EL device with a practical efficiency and lifetime.

### I-3. Luminescent material for organic EL device containing naphthacene derivative

The luminescent material for an organic EL device of the invention is characterized by containing the above naphthacene derivative of the invention.
The use of the naphthacene derivative of invention in an emitting layer of an organic EL device can realize an organic EL device with a practical efficiency and lifetime.

### II. Organic EL device

The organic EL device of the invention comprises a cathode, an anode, and one or more organic layers interposed therebetween. At least one of the organic layers contains the naphthacene derivative of the invention.

In the organic EL device of the invention, the organic layer containing the naphthacene derivatives of the invention is preferably an emitting layer. The organic EL device of the invention can have a practical efficiency and exhibit a long lifetime by the emitting layer containing the naphthacene derivatives of the invention.

The organic EL device of the invention comprises at least an emitting layer and an electron-transporting layer provided between a cathode and an anode. The emitting layer contains a host material which is the naphthacene derivative of the invention and a dopant material which is an indenoperylene derivative.

It is preferable that the indenoperylene derivative of the dopant material in the emitting layer be one or more compounds selected from the group consisting of indenoperylene derivatives represented by the following general formulas (11) to (15).

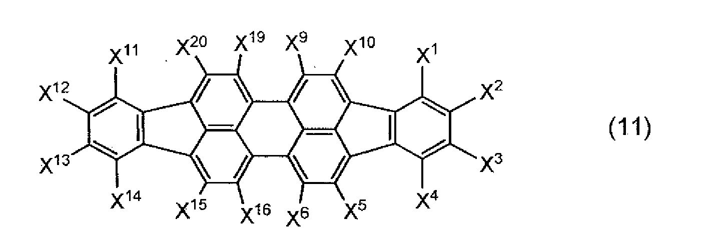

wherein X¹ to X⁶, X⁹ to X¹⁶, X¹⁹ and X²⁰ are each independently a halogen atom, alkyl group, alkoxy group, alkylthio group, alkenyl group, alkenyloxy group, alkenylthio group, aromatic-ring-containing alkyl group, aromatic-ring-containing alkyloxy group, aromatic-ring-containing alkylthio group, aromatic ring group, aromatic heterocyclic group, aromatic ring oxy group, aromatic ring thio group, aromatic ring alkenyl group, alkenyl aromatic ring group, amino group, carbazolyl group, cyano group, hydroxy group, -COOR⁵¹ (R⁵¹ is a hydrogen atom, alkyl group, alkenyl group, aromatic-ring-containing alkyl group or aromatic ring group), -COR⁵² (R⁵² is a hydrogen atom, alkyl group, alkenyl group, aromatic-ring-containing alkyl group, aromatic ring group or amino group) or -OCOR⁵³ (R⁵³ is an alkyl group, alkenyl group, aromatic-ring-containing alkyl group or aromatic ring group).

Adjacent groups of X¹ to X⁶, X⁹ to X¹⁶, X¹⁹ and X²⁰ may be bonded to each other to form a ring with a substituted carbon atom. At least one of X¹ to X⁶, X⁹ to X¹⁶, X¹⁹, and X²⁰ is not hydrogen.

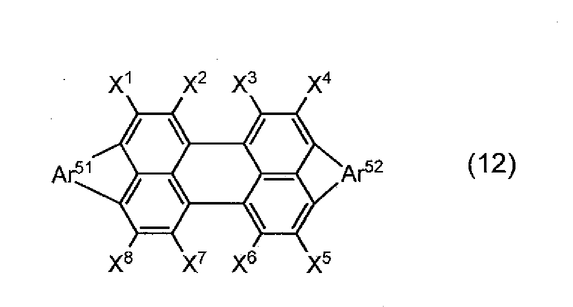

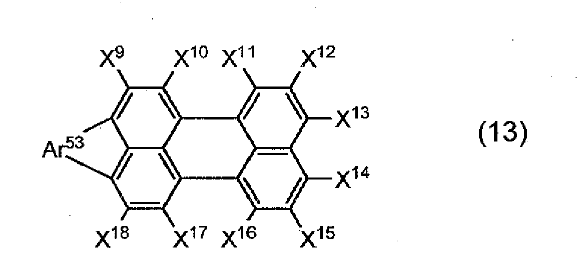

wherein Ar⁵¹, Ar⁵² and Ar⁵³ are each independently a substituted or unsubstituted aromatic hydrocarbon group having 6 to 50 ring carbon atoms, or substituted or unsubstituted aromatic heterocyclic group having 6 to 50 ring atoms.
X¹ to X¹⁸ are each independently a hydrogen atom, halogen atom, substituted or unsubstituted alkyl group having 1 to 50 carbon atoms, substituted or unsubstituted alkoxy group having 1 to 50 carbon atoms, substituted or unsubstituted alkylthio group having 1 to 50 carbon atoms, substituted or unsubstituted alkenyl group having 2 to 50 carbon atoms, substituted or unsubstituted alkenyloxy group having 1 to 50 carbon atoms, substituted or unsubstituted alkenylthio group having 1 to 50 carbon atoms, substituted or unsubstituted aromatic hydrocarbon group having 6 to 50 ring carobon atoms, substituted or unsubstituted aromatic heterocyclic group having 6 to 50 ring atoms, substituted or unsubstituted aryloxy group having 6 to 50 ring carbon atoms, substituted or unsubstituted arylthio group having 6 to 50 ring carbon atoms, substituted or unsubstituted aralkyl group having 7 to 50 ring carbon atoms, substituted or unsubstituted arylalkyloxy group having 6 to 50 ring carbon atoms, substituted or unsubstituted arylalkylthio group having 6 to 50 ring carbon atoms, substituted or unsubstituted arylalkenyl group having 6 to 50 ring carbon atoms, substituted or unsubstituted alkenylaryl group having 6 to 50 ring carbon atoms, amino group, carbazolyl group, cyano group, hydroxy group, -COOR⁵¹, -COR⁵² or -OCOR⁵³ (R⁵¹, R⁵² and R⁵³ are each a hydrogen atom, substituted or unsubstituted alkyl group having 1 to 50 carbon atoms, substituted or unsubstituted alkenyl group having 2 to 50 carbon atoms, substituted or unsubstituted aralkyl group having 7 to 50 ring carbon atoms, substituted or unsubstituted aromatic hydrocarbon group having 6 to 50 ring carbon atoms, or substituted or unsubstituted aromatic heterocyclic group having 6 to 50 ring atoms).

Adjacent groups of X¹ to X¹⁸ may be bonded to each other to form a ring with a substituted carbon atom; and at least one of X¹ to X¹⁸ is not a hydrogen atom.

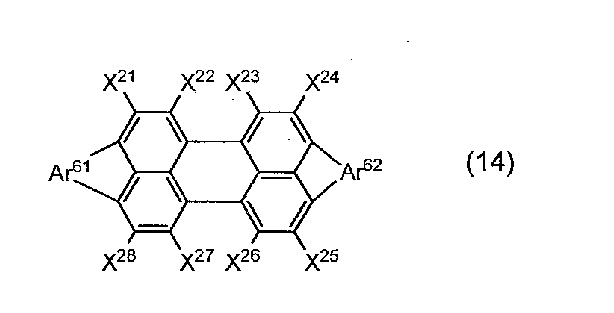

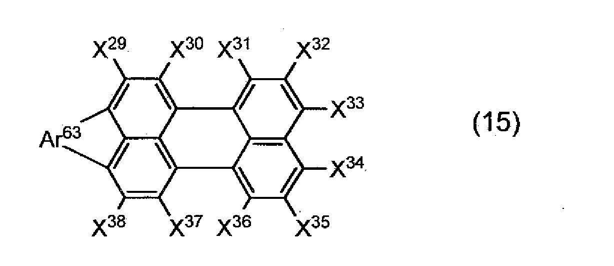

wherein Ar⁶¹, Ar⁶² and Ar⁶³ are each independently a substituted or unsubstituted aromatic hydrocarbon group having 6 to 50 ring carbon atoms, or substituted or unsubstituted aromatic heterocyclic group having 6 to 50 ring atoms.

X²¹ to X³⁸ are each independently a hydrogen atom, halogen atom, substituted or unsubstituted alkyl group having 1 to 50 carbon atoms, substituted or unsubstituted alkoxy group having 1 to 50 carbon atoms, substituted or unsubstituted alkylthio group having 1 to 50 carbon atoms, substituted or unsubstituted alkenyl group having 2 to 50 carbon atoms, substituted or unsubstituted alkenyloxy group having 1 to 50 carbon atoms, substituted or unsubstituted alkenylthio group having 1 to 50 carbon atoms, substituted or unsubstituted aromatic hydrocarbon group having 6 to 50 ring carbon atoms, substituted or unsubstituted aromatic heterocyclic group having 6 to 50 ring atoms, substituted or unsubstituted aryloxy group having 6 to 50 ring carbon atoms, substituted or unsubstituted arylthio group having 6 to 50 ring carbon atoms, substituted or unsubstituted aralkyl group having 7 to 50 ring carbon atoms, substituted or unsubstituted arylalkyloxy group having 6 to 50 ring carbon atoms, substituted or unsubstituted arylalkylthio group having 6 to 50 ring carbon atoms, substituted or unsubstituted arylalkenyl group having 6 to 50 ring carbon atoms, substituted or unsubstituted alkenylaryl group having 6 to 50 ring carbon atoms, amino group, carbazolyl group, cyano group, hydroxy group, -COOR⁵⁴, -COR⁵⁵, or - OCOR⁵⁶ (R⁵⁴, R⁵⁵ and R⁵⁶ are each a hydrogen atom, substituted or unsubstituted alkyl group having 1 to 50 carbon atoms, substituted or unsubstituted alkenyl group having 2 to 50 carbon atoms, substituted or unsubstituted aralkyl group having 7 to 50 ring carbon atoms, substituted or unsubstituted aromatic hydrocarbon group having 6 to 50 ring carbon atoms, or substituted or unsubstituted aromatic heterocyclic group having 6 to 50 ring atoms).

Adjacent groups of X²¹ to X³⁸ may be bonded to each other to form a ring with a substituted carbon atom; provided that at least one of substituents of Ar⁶¹, Ar⁶² and Ar⁶³, X²¹ to X³⁸ and substituents of X²¹ to X³⁸ is a halogen atom.

An indenoperylene derivative for a dopant material includes a dibenzotetraphenyl periflanthene derivative.
The indenoperylene derivative is preferably a dibenzotetraphenyl periflanthene derivative or the like.

A doping concentration of the indenoperylene derivative which is a dopant material is preferably 0.1 to 10 wt%, more preferably 0.5 to 2 wt%. If the doping concentration is less than 0.1 wt%, the host material may emit light. If the doping concentration is more than 10 wt%, concentration quenching may be caused.

In the organic EL device of the invention, an electron-transporting layer preferably contains a compound represented by the following general formula (5)

A-B (5)

wherein A is an aromatic hydrocarbon group with 3 or more carbocircles and B is a heterocyclic group which may be substituted.

The aromatic hydrocarbon group with 3 or more carbocircles of the group A includes anthracene, phenanthrene, naphthacene, pyrene, chrysene, benzanthracene, pentacene, dibenzoanthracene, benzopyrene, fluorene, benzofluorene, fluoranthene, benzofluoranthene, naphthofluoranthene, dibenzofluorene, dibenzopyrene and dibenzofluoranthene.

The substituted or unsubstituted heterocyclic group of the group B includes pyridine, pyrimidine, pyrazine, pyridazine, triazine, quinoline, quinoxaline, acridine, imidazopyridine, imidazopyrimidine and phenenthroline.

The compound represented by formula (5) is preferably a compound containing in the molecule thereof at least one skeleton selected from anthracene, phenanthrene, naphthacene, pyrene, chrysene, benzoanthracene, pentacene, dibenzoanthracene, benzopyrene, fluorene, benzofluorene, fluoranthene, benzofluoranthene, naphthofluoranthene, dibenzofluorene, dibenzopyrene and dibenzofluoranthene.

The compound represented by the general formula (5) is preferably a nitrogen-containing heterocyclic compound.
The nitrogen-containing heterocyclic compound preferably contains in the molecule thereof at least one skeleton selected from pyridine, pyrimidine, pyrazine, pyridazine, triazine, quinoline, quinoxaline, acridine, imidazopyridine, imidazopyrimidine and phenenthroline.

The nitrogen-containing heterocyclic compound is more preferably a benzoimidazole derivative represented by the following general formula (6) or (7).

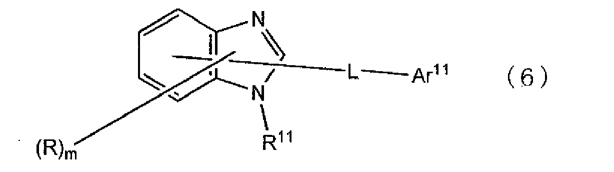

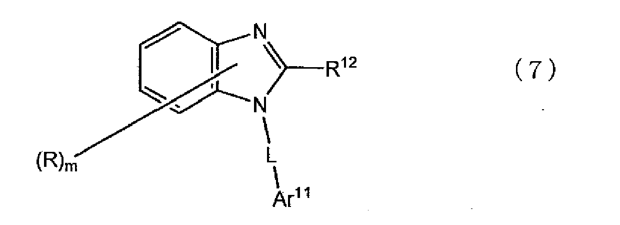

wherein R is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 50 carbon atoms or substituted or unsubstituted alkoxy group having 1 to 50 carbon atoms.
As examples of the aryl group having 6 to 60 carbon atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphtyl group, 4-methyl-1-naphtyl group, 4-methyl-1-anthryl group, 4'-methylbiphenylyl group, 4''-t-butyl-p-terphenyl-4-yl group, fluoranthenyl group, fluorenyl group and the like are preferable, and a phenyl group, naphthyl group, biphenyl group, anthracenyl group, phenanthryl group, pyrenyl group, crycenyl group, fluoranthenyl group and fluorenyl group and the like are more preferable.

As examples of the alkyl group having 1 to 50 carbon atoms, a methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminomethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group, 2-norbornyl group, and the like are preferable.
The alkoxy group having 1 to 50 carbon atoms is represented by -OY. Examples of Y include the same groups as the above-mentioned examples for the alkoxy group.

As a substituent of the above aryl group, pyridinyl group, quinolinyl group, alkyl group or alkoxy group, a substituted or unsubstituted aryl group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 1 to 50 carbon atoms, a substituted or unsubstituted aryloxy group having 5 to 50 ring atoms, a substituted or unsubstituted arylthio group having 5 to 50 ring atoms, a substituted or unsubstituted carboxyl group having 1 to 50 carbon atoms, a halogen group, a cyano group, a nitro group, a hydroxy group, or the like are given.

m is an integer from 0 to 4, preferably from 0 to 3 and more preferably from 0 to 2.

R¹¹ is a substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms or alkoxy group having 1 to 20 carbon atoms.
Examples of the groups and substituents of R¹¹ are the same as those of the above-mentioned R.

R¹² is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or substituted or unsubstituted alkoxy group having 1 to 20 carbon atoms. Examples of the groups and substituents of R¹² are the same as those of the above-mentioned R.

L is a substituted or unsubstituted arylene group having 6 to 60 carbon atoms, substituted or unsubstituted pyridinylene group, substituted or unsubstituted quinoliylene group, or substituted or unsubstituted fluorenylene group.
The arylene group having 6 to 60 carbon atoms is preferably a divalent substituent obtainable by removing one hydrogen atom from the substituent described for the aryl group having 6 to 60 carbon atoms, more preferably a phenylene group, naphthylene group, biphenylene group, anthracenylene group, phenanthrylene group, pyrenylene group, chrysenylene group, fluoranthenylene group or fluorenylene group.

Examples of substituents of the arylene group, pyridinylene group, quinoliylene group or fluorenylene group are the same as those of the above-mentioned R.

Ar¹¹ is a substituted or unsubstituted aryl group having 6 to 60 carbon atoms, a substituted or unsubstituted pyridinyl group or a substituted or unsubstituted quinolinyl group.
Substituents of the aryl group having 6 to 60 carbon atoms, pyridinyl group and quinolinyl group are the same as those of the above-menioned R.

For the benzoimidazole derivatives represented by formulas (6), m is preferably 0, R¹¹ is preferably an aryl group, L is preferably an arylane group with 6 to 30 carbon atoms (more preferably 6 to 20 carbon atoms) and Ar¹¹ is preferably an aryl group with 6 to 30 carbon atoms.

For the benzoimidazole derivatives represented by formulas (7), m is preferably 0, R¹² is preferably an aryl group, L is preferably an arylane group with 6 to 30 carbon atoms (more preferably 6 to 20 carbon atoms) and Ar¹¹ is preferably an aryl group with 6 to 30 carbon atoms.

The structure of the organic EL device of the invention will be described with reference to FIG. 1.
FIG. 1 is a cross-sectional view showing an example of the organic EL device according to the invention.
An organic EL device 1 has a configuration in which an anode 20, a hole injecting layer 30, a hole transporting layer 40, an emitting layer 50, an electron transporting layer 60, an electron injecting layer 70, and a cathode 80 are stacked on a substrate 10 in that order. The organic EL device can have the other structures.

### [Structure of organic EL device]

The typical examples of the structure of the organic EL device of the invention are shown below. The invention is not limited to these.
(1) Anode/emitting layer/electron-transporting layer/hole-transporting layer/cathode
(2) Anode/hole-transporting layer/emitting layer/electron-transporting layer/cathode
(3) Anode/hole-injecting layer/hole-transporting layer/emitting layer/electron-transporting layer/cathode
(4) Anode/hole-transporting layer/emitting layer/electron-transporting layer/electron-injecting layer/cathode
(5) Anode/hole-injecting layer/hole-transporting layer/emitting layer/eleetron-transporting layer/electron-injecting layer/cathode
(6) Anode/insulating layer/hole-transporting layer/emitting layer/electron-transporting layer/cathode
(7) Anode/hole-transporting layer/emitting layer/electron-transporting layer/insulating layer/cathode
(8) Anode/insulating layer/hole-transporting layer/emitting layer/electron-transporting layer/insulating layer/cathode
(9) Anode/hole-injecting layer/hole-transporting layer/emitting layer/electron-transporting layer/insulating layer/cathode
(10) Anode/insulating layer/hole-injecting layer/hole-transporting layer/emitting layer/electron-transporting layer/electron-injecting layer/cathode
(11) Anode/insulating layer/hole-injecting layer/hole-transporting layer/emitting layer/electron-transporting layer/electron-injecting layer/insulating layer/cathode
   Among these, the structures (2), (3), (4), (5), (8), (9) and (11) are generally preferably used.

Functions of individual layers of an organic EL device are described below.

### [Transparent substrate]

If an organic EL device is of under surface emission type or bottom emission type where light is outcoupled through a substrate, the organic EL device of the invention is formed on transparent substrate. The transparent substrate is a substrate for supporting the organic EL device, and is preferably a flat and smooth substrate having a transmittance of 50% or more to light rays within visible ranges of 400 to 700 nm.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfide, and polysulfone. A TFT substrate where TFT is formed for drive may be used.

If an organic EL device is of upper surface emission type or top emission type where light is outcoupled from the upper part of the device, a light-reflecting metal such as aluminum is provided on the above substrate.

### [Anode]

The anode of the organic EL device of the invention plays a role for injecting holes into its hole-transporting layer or emitting layer. The anode effectively has a work function of 4.5 eV or more. Specific examples of the material of the anode used in the invention include indium tin oxide alloy (ITO), zinc tin oxide alloy (IZO), tin oxide (NESA), gold, silver, platinum, and copper.
Although these materials may be used individually, alloys thereof or materials wherein another element is added to the materials can be selected for use.
The anode can be formed by forming these electrode materials into a thin film by vapor deposition, sputtering or the like.

In an organic EL device of under sueface emission or bottom emission type, an anode preferably has a transmittance of 10 % or more to emitted light. The sheet resistance of the anode is preferably several hundreds Ω/□ or less. The film thickness of the anode, which varies depending upon the material thereof, is usually from 10 nm to 1 µm, preferably from 10 to 200 nm.

### [Emitting layer]

The emitting layer of the organic EL device has the following functions in combination. Namely,
(i) Injecting function: function of allowing injection of holes from anode or hole injecting/transporting layer and injection of electrons from cathode or electron injecting/transporting layer upon application of electric field
(ii) Transporting function: function of moving injected carriers (electrons and holes) due to force of electric field
(iii) Emitting function: function of providing a site for recombination of electrons and holes to emit light
   Note that electrons and holes may be injected into the emitting layer with different degrees, or the transportation capabilities indicated by the mobilities of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film.
The term "molecular deposition film" refers to a thin film formed by depositing a vapor-phase material compound or a film formed by solidifying a solution-state or liquid-phase material compound. The molecular deposition film is distinguished from a thin film (molecular accumulation film) formed using the LB method by the difference in aggregation structure or higher order structure or the difference in function due to the difference in structure.
The emitting layer may also be formed by dissolving a binder such as a resin and a material compound in a solvent to obtain a solution, and forming a thin film from the solution by spin coating or the like, as disclosed in JP-A-57-51781.

### [Hole-transporting and hole-injecting layer]

The hole-transporting layer is a layer for helping the injection of holes into the emitting layer so as to transport holes to an emitting region. The hole mobility thereof is large and the ionization energy thereof is usually as small as 5.5 eV or less. Such a hole-transporting layer is preferably made of a material which can transport holes to the emitting layer at a low electric field intensity. The hole mobility thereof is preferably at least 10⁻⁴ cm²/V·second when an electric field of, e.g., 10⁴ to 10⁶ V/cm is applied.
Any materials which have the above preferable properties can be used as the material for forming the hole-transporting layer without particular limitation. The material for forming the hole-transporting layer can be arbitrarily selected from materials which have been widely used as a material transporting carriers of holes in photoconductive materials and known materials used in a hole-transporting layer of EL devices.

Specific examples include triazole derivatives (see USP No. 3,112,197 and others), oxadiazole derivatives (see USP No. 3,189,447 and others), imidazole derivatives (see JP-B-37-16096 and others), polyarylalkane derivatives (see USP Nos. 3,615,402, 3,820,989 and 3,542,544, JP-B-45-555 and 51-10983, JP-A-51-93224, 55-17105, 56-4148, 55-108667, 55-156953 and 56-36656, and others), pyrazoline derivatives and pyrazolone derivatives (see USP Nos. 3,180,729 and 4,278,746, JP-A-55-88064, 55-88065, 49-105537, 55-51086, 56-80051, 56-88141, 57-45545, 54-112637 and 55-74546, and others), phenylene diamine derivatives (see USP No. 3,615,404, JP-B-51-10105, 46-3712 and 47-25336, JP-A-54-53435, 54-110536 and 54-119925, and others), arylamine derivatives (see USP Nos. 3,567,450, 3,180,703, 3,240,597, 3,658,520, 4,232,103, 4,175,961 and 4,012,376, JP-B-49-35702 and 39-27577, JP-A-55-144250, 56-119132 and 56-22437, DE1,110,518, and others), amino-substituted chalcone derivatives (see USP No. 3,526,501, and others), oxazole derivatives (ones disclosed in USP No. 3,257,203, and others), styrylanthracene derivatives (see JP-A-56-46234, and others), fluorenone derivatives (JP-A-54-110837, and others), hydrazone derivatives (see USP Nos. 3,717,462, JP-A-54-59143, 55-52063, 55-52064, 55-46760, 55-85495, 57-11350, 57-148749 and 2-311591, and others), stilbene derivatives (see JP-A-61-210363, 61-228451, 61-14642, 61-72255, 62-47646, 62-36674, 62-10652, 62-30255, 60-93455, 60-94462, 60-174749 and 60-175052, and others), silazane derivatives (USP No. 4,950,950), polysilanes (JP-A-2-204996), aniline copolymers (JP-A-2-282263), and electroconductive high molecular oligomers (in particular thiophene oligomers) disclosed in JP-A-1-211399.

The material shown in formula (8) below is preferably used.

Q¹-G-Q² (8)

wherein Q¹ and Q² are parts having at least one tertiary amine and G is a linkage group.)

More preferably, an amine derivative shown by formula (9) below is used. 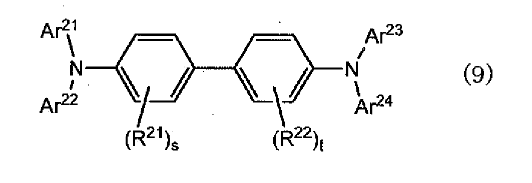 wherein Ar²¹ to Ar²⁴ are a substituted or unsubstituted aromatic ring having 6 to 50 ring carbon atoms, or substituted or unsubstituted aromatic heterocycle having 5 to 50 ring atoms.
R²¹ and R²² are substituents and s and t are integers from 0 to 4, respectively.
Ar²¹ and Ar²², and Ar²³ and Ar²⁴ may be bonded together to form a ring, respectively.
R²¹ and R²² may also be bonded together to form a ring.
Substituents of Ar²¹ to Ar²⁴, R²¹ and R²² are a substituted or unsubstituted aromatic ring having 6 to 50 ring carbon atoms, substituted or unsubstituted aromatic heterocycle having 5 to 50 ring atoms, alkyl group having 1 to 50 carbon atoms, alkoxy group having 1 to 50 carbon atoms, alkylaryl group having 1 to 50 carbon atoms, aralkyl group having 1 to 50 carbon atoms, styryl group, amino group substituted with an aromatic ring having 6 to 50 ring carbon atoms or a hetero aromatic ring having 5 to 50 ring atoms, or aromatic ring having 6 to 50 ring carbon atoms or hetero aromatic ring having 5 to 50 ring atoms, the aromatic ring and the hetero aromatic ring being substituted with an amino group substituted with an aromatic ring having 6 to 50 ring carbon atoms or a hetero aromatic ring having 5 to 50 ring atoms.

Further, a hole-injecting layer can be provided in addition to the hole-transporting layer so as to help the injection of holes. The same substances used for the hole-transporting layer can be used as the material of the hole-injecting layer. The following can also be used: porphyrin compounds (disclosed in JP-A-63-2956965 and others), aromatic tertiary amine compounds and styrylamine compounds (see USP No. 4,127,412, JP-A-53-27033, 54-58445, 54-149634, 54-64299, 55-79450, 55-144250, 56-119132, 61-295558, 61-98353 and 63-295695, and others). Aromatic tertiary amine compounds are particularly preferably used.

The following can also be given as examples: 4,4'-bis(N-(1-naphthyl)-N-phenylamino)biphenyl (abbreviated as NPD hereinafter), which has in the molecule thereof two condensed aromatic rings, disclosed in USP No. 5,061,569, and 4,4',4"-tris(N-(3-methylphenyl)-N-phenylamino)triphenylamine (abbreviated as MTDATA, hereinafter), wherein three triphenylamine units are linked to each other in a star-burst form, disclosed in JP-A-4-308688.
Inorganic compounds such as p-type Si and p-type SiC as well as aromatic dimethylidene type compounds can also be used as the material of the hole-transporting layer.

The hole-injecting layer and hole-transporting layer can be formed by making the above-mentioned compound(s) into a thin film by a known method such as vacuum deposition, spin coating, casting or LB technique. The film thickness of the hole-injecting layer and hole-transporting layer is not particularly limited, and is usually from 5 nm to 5 µm. This hole-injecting layer and hole-transporting layer may be a single layer made of one or two or more of the above-mentioned materials so far as this layer contains the above-mentioned compound in a hole-transporting zone. Otherwise they may be stacked hole-injecting and hole-transporting layers made of different compounds.

An organic semiconductor layer is one type of a hole transporting layer for helping the injection of holes or electrons into an emitting layer, and is preferably a layer having an electric conductivity of 10⁻¹⁰ S/cm or more. As the material of such an organic semiconductor layer, electroconductive oligomers such as thiophene-containing oligomers or arylamine-containing oligomers disclosed in JP-A-8-193191, and electroconductive dendrimers such as arylamine-containing dendrimers may be used.

### [Electron-injecting layer]

The electron-injecting layer is a layer for helping the injection of electrons into an emitting layer, and has a large electron mobility. An adhesion improving layer is a layer made of a material particularly good in adhesion to a cathode among such electron-injecting layers.

A preferred embodiment of the invention is a device containing a reducing dopant in an interfacial region between its electron transferring region or cathode and organic layer. The reducing dopant is defined as a substance which can reduce an electron transferring compound. Accordingly, various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.

More specific examples of the preferred reducing dopants include at least one alkali metal selected from the group consisting of Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). Metals having a work function of 2.9 eV or less are particularly preferred. Among these, a more preferable reducing dopant is at least one alkali metal selected from the group consisting of K, Rb and Cs. Even more preferable is Rb or Cs. Most preferable is Cs. These alkali metals are particularly high in reducing ability. Thus, the addition of a relatively small amount thereof to an electron injecting zone improves the luminance of the organic EL device and make the lifetime thereof long. As the reducing dopant having a work function of 2.9 eV or less, a combination of two or more out of these alkali metals is also preferred. Particularly preferred is a combination containing Cs, for example, combinations of Cs and Na, Cs and K, Cs and Rb, or Cs, Na and K. The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By providing the layer, current leakage can be effectively prevented to improve the injection of electrons. As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved. Specifically preferable alkali metal calcogenides include Li₂O, LiO, Na₂S, Na₂Se and NaO and preferable alkaline earth metal calcogenides include CaO, BaO, SrO, BeO, BaS and CaSe. Preferable halides of alkali metals include LiF, NaF, KF, LiCl, KCl and NaCl. Preferable halides of alkaline earth metals include fluorides such as CaF₂, BaF₂, SrF₂, MgF₂ and BeF₂ and halides other than fluorides.

Examples of the semiconductor for forming an electron-injecting layer include oxides, nitrides or oxynitrides containing at least one element selected from Ba, Ca, Sr, Yb, Al, Ga, In, Li, Na, Cd, Mg, Si, Ta, Sb and Zn, and combinations of two or more thereof. The inorganic compound for forming an electron-injecting layer is preferably a microcrystalline or amorphous insulating thin film. When an electron-injecting layer is formed of the insulating thin film, a more uniform thin film can be formed to reduce pixel defects such as dark spots.
Examples of such an inorganic compound include the above-mentioned alkali metal calcogenides, alkaline earth metal calcogenides, halides of alkali metals, and halides of alkaline earth metals.

### [Cathode]

For the cathode, in order to inject electrons to an electron injecting/transporting layer or emitting layer, the following electrode substance may be used: metals, alloys or electroconductive compounds, or mixtures thereof which have a small work function (4 eV or less). Specific examples of the electrode substance include sodium, sodium-potassium alloy, magnesium, lithium, magnesium/silver alloy, aluminum/aluminum oxide, aluminum/lithium alloy, indium, and rare earth metals.

This cathode can be formed by making the electrode substances into a thin film by vapor deposition, sputtering or some other method.
In organic EL device of upper surface emission or top emission type, a cathode preferably has a transmittance of 10% or more to emitted light.
The sheet resistance of the cathode is preferably several hundreds Ω/□ or less, and the film thickness thereof is usually from 10 nm to 1 µm, preferably from 50 to 200 nm.

### [Insulating layer]

In the organic EL device, pixel defects due to leakage or a short circuit are easily generated since an electric field is applied to super thin films. In order to prevent this, it is preferred to insert an insulator thin layer between a pair of electrodes.

Examples of the material used in the insulating layer include aluminum oxide, lithium fluoride, lithium oxide, cesium fluoride, cesium oxide, magnesium oxide, magnesium fluoride, calcium oxide, calcium fluoride, cesium fluoride, cesium carbonate, aluminum nitride, titanium oxide, silicon oxide, germanium oxide, silicon nitride, boron nitride, molybdenum oxide, ruthenium oxide, and vanadium oxide.
A mixture or laminate thereof may be used.

### [Example of fabricating organic EL device]

The organic EL device can be fabricated by forming an anode, an emitting layer, optionally forming a hole-injecting layer, a hole-transporting layer, an electron-transporting layer and an electron-injecting layer if necessary, and further forming a cathode by use of the materials and methods exemplified above. The organic EL device can be fabricated in the order reverse to the above, i.e., the order from a cathode to an anode.

An example of the fabrication of the organic EL device will be described below which has a structure wherein the following are successively formed on a transparent substrate: anode/hole-transporting layer/emitting layer/electron-transporting layer/cathode.
First, a thin film made of an anode material is formed into a thickness of 1 µm or less, preferably 10 to 200 nm on an appropriate transparent substrate by vapor deposition, sputtering or some other method, thereby forming an anode. Next, a hole-transporting layer is formed on this anode. As described above, the hole-transporting layer can be formed by vacuum deposition, spin coating, casting, LB technique, or some other method. Vacuum deposition is preferred since a homogenous film is easily obtained and pinholes are not easily generated. In the case where the hole-transporting layer is formed by vacuum deposition, conditions for the deposition vary depending upon a compound used (a material for the hole-transporting layer), a desired crystal structure or recombining structure of the hole-transporting layer, and others. In general, the conditions are preferably selected from the following: deposition source temperature of 50 to 450°C, vacuum degree of 10⁻⁷ to 10⁻³ torr, vapor deposition rate of 0.01 to 50 nm/second, substrate temperature of -50 to 300°C, and film thickness of 5 nm to 5 µm.

Next, an emitting layer is formed on the hole-transporting layer. The emitting layer can also be formed by making a desired organic luminescent material into a thin film by vacuum deposition, sputtering, spin coating, casting or some other method. Vacuum deposition is preferred since a homogenous film is easily obtained and pinholes are not easily generated. In the case where the emitting layer is formed by vacuum deposition, conditions for the deposition, which vary depending on a compound used, can be generally selected from conditions similar to those for the hole-transporting layer.

Next, an electron-transporting layer is formed on this emitting layer. Like the hole-transporting layer and the emitting layer, the layer is preferably formed by vacuum deposition because a homogenous film is required. Conditions for the deposition can be selected from conditions similar to those for the hole-transporting layer and the emitting layer.
Lastly, a cathode is stacked thereon to obtain an organic EL device.

The cathode is made of a metal, and vapor deposition or sputtering may be used. However, vacuum deposition is preferred in order to protect underlying organic layers from being damaged when the cathode film is formed.
For the organic EL device fabrication that has been described above, it is preferred that the formation from the anode to the cathode is continuously carried out, using only one vacuuming operation.

The method for forming each of the layers in the organic EL device of the invention is not particularly limited. A known forming method, such as vacuum deposition, molecular beam deposition, spin coating, dipping, casting, bar coating or roll coating can be used.

The film thickness of each of the organic layers in the organic EL device of the invention is not particularly limited. In general, defects such as pinholes are easily generated when the film thickness is too small. Conversely, when the film thickness is too large, a high applied voltage becomes necessary, leading to low efficiency. Usually, the film thickness is preferably in the range of several nanometers to one micrometer. If a voltage is applied to the organic EL device, emission can be observed when the polarities of the anode and the cathode are positive and negative, respectively, and a voltage of 3 to 40 V is applied. When a voltage with an opposite polarity is applied, no electric current flows and hence, emission does not occur. If an AC voltage is applied, uniform emission can be observed only when the cathode and the anode have a positive polarity and a negative polarity, respectively. The waveform of the AC applied may be arbitrary.

### Examples

The invention is described specifically using examples. The invention is not limited to these examples.

### Synthesis Example 1

### Synthesis of compounds A-1 and A-2

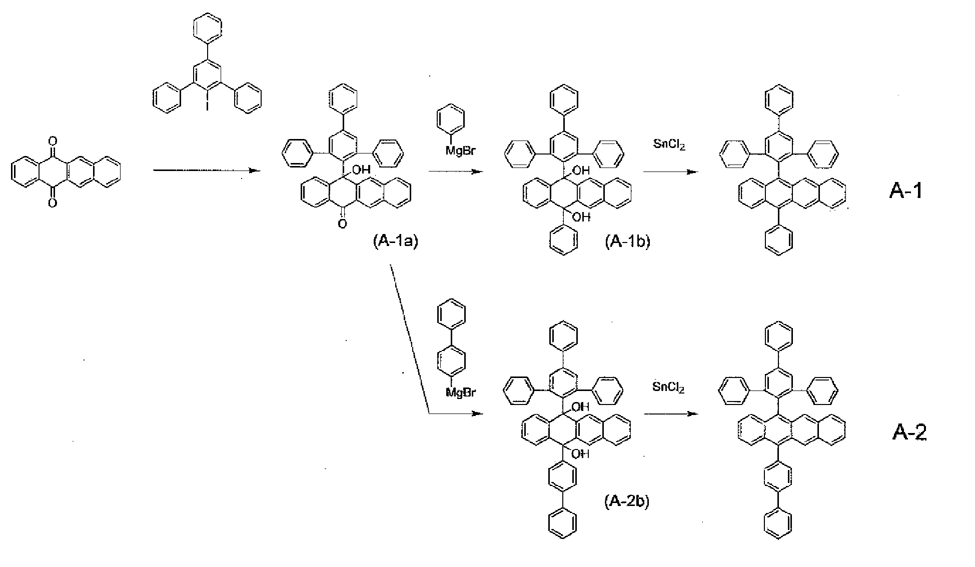

### (1) Synthesis of intermediate (A-1a)

5.5 g of 2,4,6-triphenyliodobenzene was dissolved in 40 ml of toluene. After adding 13 mL of diethyl ether, the resultant mixture was cooled to -55°C. 8 mL of a 1.6 M solution of n-butyl lithium in n-hexane was added, and the resultant mixture was stirred for one hour. 2.6 g of 5,12-naphthacene quinone powder was added, and then the resultant mixture was allowed to react for three hours with gradually increasing the temperature to 0°C. The reaction was stopped by adding 20 mL of methanol, and the solid produced was recovered by filtering, followed by washing with methanol. The resultant solid was purified by silicagel column chromatography to obtain 5.7 g (yield: 99%) of pale yellow powder of an intended intermediate (A-1a).

### (2) Synthesis of intermediate (A-1b)

2.8 g of the intermediate (A-1a) was dissolved in 30 mL of tetrahydrofuran, and 15 mL of a 1 M solution of phenyl magnesium bromide in tetrahydrofuran was dripped into the solution at room temperature for 10 minutes. Then, the resultant mixture was heated to reflux for 1 hour to finish the reaction. After cooling the reaction mixture to room temperature, an aquous ammonium chloride solution was added to stop the reaction. The reaction mixture was extracted using diethyl ether, and the solvent was evaporated under reduced pressure. After adding methanol to the residue, precipitated powder was filtered out, and was washed with methanol. After washing the powder with toluene under heated reflux, the resultant powder was dried under vacuum to obtain 2.2 g (yield: 68%) of white powder of an intended intermediate (A-1b).

### (3) Synthesis of compound A-1

80 mL of tetrahydrofuran was added to 2.2 g of the intermediate (A-1b) and the mixture was heated to about 40°C to dissolve the intermediate under blocking light. 28 mL of an aquous concentrated hydrochloric acid solution containing 7.7 g of tin chloride dihydrate was dripped into the solution for 30 minutes, and the resultant mixture was heated to reflux for 2 hours to finish the reaction. After cooling the reaction mixture to room temperature, 200 mL of distilled water was added, and the powder produced was filtered out, followed by washing with methanol. The powder was dried under vacuum to obtain 2.0 g (yield: 98%) of yellow orange powder of an intended compound A-1. For the compound, sublimation and purification were performed twice under reduced pressure, and the compound obtained was used for a device production.

### (4) Synthesis of intermediate (A-2b)

2.8 g of the intermediate (A-1a) was dissolved in 30 mL of tetrahydrofuran, and 15 mL of a 1 M solution of p-biphenyl magnesium bromide in tetrahydrofuran was dripped into the solution at room temperature for 10 minutes. Then, the resultant mixture was heated to reflux for 1 hour to finish the reaction. After cooling the reaction mixture to room temperature, an aquous ammonium chloride solution was added to stop the reaction. The reaction mixture was extracted using diethyl ether, and the solvent was evaporated under reduced pressure. After adding methanol to the residue, precipitated powder was filtered out, and was washed with methanol. After washing the powder with toluene under heated reflux, the resultant powder was dried under vacuum to obtain 2.5 g (yield: 70%) of white powder of an intended intermediate (A-2b).

### (5) Synthesis of compound A-2

80 mL of tetrahydrofuran was added to 2.4 g of the intermediate (A-2b) and the mixture was heated to about 40°C to dissolve the intermediate under blocking light. 28 mL of an aquous concentrated hydrochloric acid solution containing 7.7 g of tin chloride dihydrate was dripped into the solution for 30 minutes, and the resultant mixture was heated to reflux for 2 hours to finish the reaction. After cooling the reaction mixture to room temperature, 200 mL of distilled water was added, and the powder produced was filtered out, followed by washing with methanol. The powder was dried under vacuum to obtain 2.2 g (yield: 96%) of yellow orange powder of an intended compound A-1. For the compound, sublimation and purification were performed twice under reduced pressure, and the compound obtained was used for a device production.

### Synthesis Example 2

### Synthesis of compound A-3

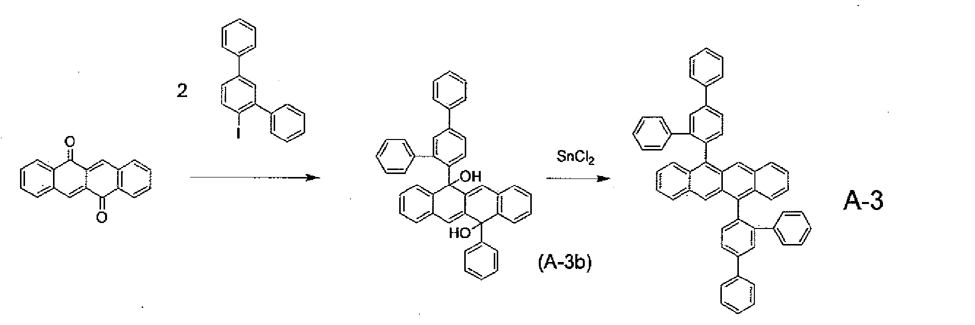

### (1) Synthesis of intermediate (A-3b)

7.4 g of 2,4-diphenyliodobenzene was dissolved in 45 ml of toluene. After adding 15 mL of diethyl ether, the resultant mixture was cooled to -55°C. 13 mL of a 1.6 M solution of n-butyl lithium in n-hexane was added, and the resultant mixture was stirred for one hour. 1.9 g of 5,11-naphthacene quinone powder was added, and then the resultant mixture was allowed to react for three hours with gradually increasing the temperature to 0°C. The reaction was stopped by adding 20 mL of methanol, and the solid produced was recovered by filtering, followed by washing with methanol. After washing the resultant solid with toluene under heated reflux, the resultant solid was dried under vacuum to obtain 3.3 g (yield: 60%) of pale yellow powder of an intended intermediate (A-3b).

### (2) Synthesis of compound A-3

80 mL of tetrahydrofuran was added to 3.3 g of the intermediate (A-3b) and the mixture was heated to about 40°C to dissolve the intermediate under blocking light. 28 mL of an aquous concentrated hydrochloric acid solution containing 7.7 g of tin chloride dihydrate was dripped into the solution for 30 minutes, and the resultant mixture was heated to reflux for 2 hours to finish the reaction. After cooling the reaction mixture to room temperature, 200 mL of distilled water was added, and the powder produced was filtered out, followed by washing with methanol. The powder was dried under vacuum to obtain 3.0 g (yield: 95%) of yellow orange powder of an intended compound A-3. For the compound, sublimation and purification were performed twice under reduced pressure, and the compound obtained was used for a device production.

### Synthesis Example 3

### Synthesis of compound A-4

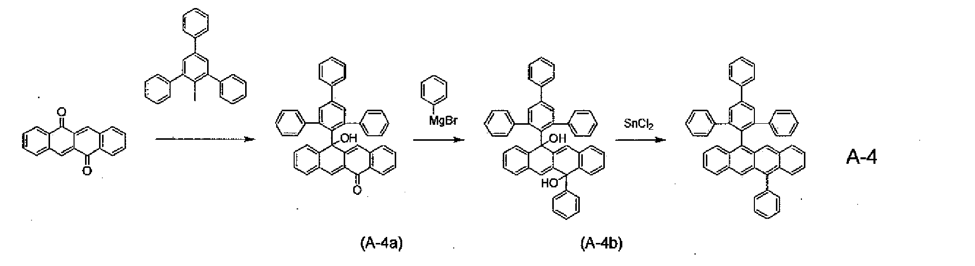

### (1) Synthesis of intermediate (A-4a)

5.5 g of 2,4,6-triphenyliodobenzene was dissolved in 40 ml of toluene. After adding 13 mL of diethyl ether, the resultant mixture was cooled to -55°C. 8 mL of a 1.6 M solution of n-butyl lithium in n-hexane was added, and the resultant mixture was stirred for one hour. 2.6 g of 5,11-naphthacene quinone powder was added, and then the resultant mixture was allowed to react for three hours with gradually increasing the temperature to 0°C. The reaction was stopped by adding 20 mL of methanol, and the solid produced was recovered by filtering, followed by washing with methanol. The resultant solid was purified by silicagel column chromatography to obtain 5.7 g (yield: 99%) of pale yellow powder of an intended intermediate (A-4a).

### (2) Synthesis of intermediate (A-4b)

2.8 g of the intermediate (A-4a) was dissolved in 30 mL of tetrahydrofuran, and 15 mL of a 1 M solution of phenyl magnesium bromide in tetrahydrofuran was dripped into the solution at room temperature for 10 minutes. Then, the resultant mixture was heated to reflux for 1 hour to finish the reaction. After cooling the reaction mixture to room temperature, an aquous ammonium chloride solution was added to stop the reaction. The reaction mixture was extracted using diethyl ether, and the solvent was evaporated under reduced pressure. After adding methanol to the residue, precipitated powder was filtered out, and was washed with methanol. After washing the powder with toluene under heated reflux, the resultant powder was dried under vacuum to obtain 2.3 g (yield: 72%) of white powder of an intended intermediate (A-4b).

### (3) Synthesis of compound A-4

80 mL of tetrahydrofuran was added to 2.3 g of the intermediate (A-4b) and the mixture was heated to about 40°C to dissolve the intermediate under blocking light. 28 mL of an aquous concentrated hydrochloric acid solution containing 7.7 g of tin chloride dihydrate was dripped into the solution for 30 minutes, and the resultant mixture was heated to reflux for 2 hours to finish the reaction. After cooling the reaction mixture to room temperature, 200 mL of distilled water was added, and the powder produced was filtered out, followed by washing with methanol. The powder was dried under vacuum to obtain 2.0 g (yield: 95%) of yellow orange powder of an intended compound A-4. For the compound, sublimation and purification were performed twice under reduced pressure, and the compound obtained was used for a device production.

### Example 1

A transparent electrode made of an indium tin oxide with a thickness of 120 nm was provided on a grass substrate measuring 25 mm by 75 mm by 0.7 mm. The grass substrate was subjected to ultrasonic cleaning with isopropyl alcohol for 5 minutes, and cleaned with ultraviolet ozone for 30 minutes. The resultant substrate was mounted in a vacuum deposition device.
N',N''-bis[4-(diphenylamino)phenyl]-N',N''-diphenylbiphenyl-4,4'-diamine was deposited to form a 60 nm thick film as an hole-injecting layer on the substrate. Thereafter N,N'-bis[4'-{N-(naphthyl-1-yl)-N-phenyl}aminobiphenyl-4-yl]-N-phenylamine was deposited to form a 10 nm thick film as a hole-transporting layer thereon. Next, the compound (A-1) of a naphthacene derivative shown below and the compound (B) of an indenoperylene derivative shown below were co-deposited such that the weight ratio of A-1 to B-1 was 40 to 0.4, to form a 40 nm thick film as an emitting layer.

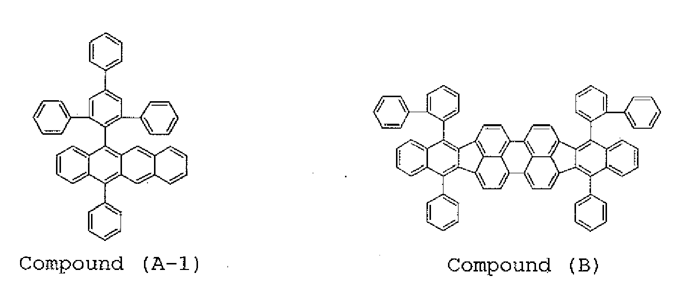

Next, the compound (C) was deposited to form a 30 nm thick film as an electron-transporting layer.

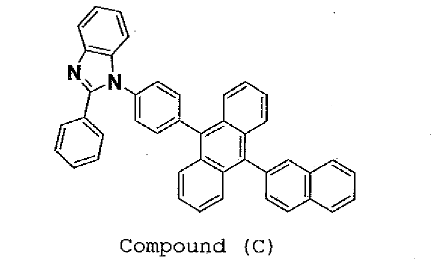

Next, lithium fluoride was deposited to form a 0.3 nm thick film, and then aluminum was deposited to form a 150 nm thick film. This aluminum/lithium fluoride functioned as a cathode. An organic EL device was thus fabricated.

For the device thus obtained, a current test was performed. Red emission with a driving voltage of 3.9 V and luminance of 1184 cd/m² was obtained at a current density of 10 mA/cm². The chromaticity coordinates were (0.67, 0.33) and the efficiency was 11.84 cd/A. A direct current continuous test was performed at an initial luminance of 5000 cd/m², and a period of time until the luminance reached 80% of the initial luminance was 2900 hours.

### Example 2

An organic EL device was fabricated in the same manner as in Example 1 except that the compound (A-2) of a naphthacene derivative, was used instead of the compound (A-1) when an emitting layer was formed.

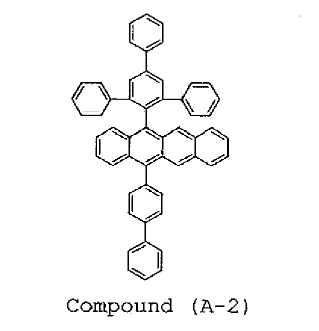

For the device thus obtained, a current test was performed. Red emission with a driving voltage of 4.0 V and luminance of 1128 cd/m² was obtained at a current density of 10 mA/cm². The chromaticity coordinates were (0.67, 0.33) and the efficiency was 11.28 cd/A. When a direct current continuous test was performed at an initial luminance of 5,000 cd/m², a half life was 3,400 hours.

### Example 3

An organic EL device was fabricated in the same manner as in Example 1 except that the compound (A-3) of a naphthacene derivative was used instead of the compound (A-1) when an emitting layer was formed.

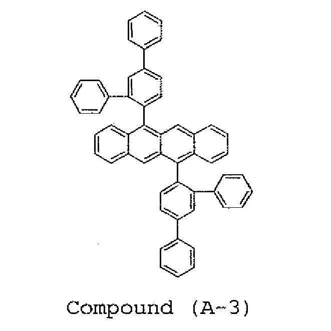

For the device thus obtained, a current test was performed. Red emission with a driving voltage of 3.8 V and luminance of 1212 cd/m² was obtained at a current density of 10 mA/cm². The chromaticity coordinates were (0.67, 0.33) and the efficiency was 12.12 cd/A. When a direct current continuous test was performed at an initial luminance of 5,000 cd/m², a half life was 3,500 hours.

### Example 4

An organic EL device was fabricated in the same manner as in Example 1 except that the compound (A-4) of a naphthacene derivative was used instead of the compound (A-1) when an emitting layer was formed.

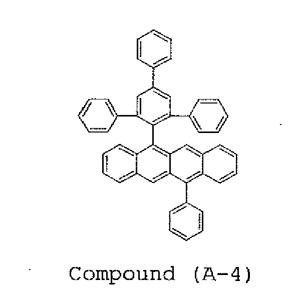

For the device thus obtained, a current test was performed. Red emission with a driving voltage of 4.0 V and luminance of 1030 cd/m² was obtained at a current density of 10 mA/cm². The chromaticity coordinates were (0.67, 0.33) and the efficiency was 11.06 cd/A. A direct current continuous test was performed at an initial luminance of 5000 cd/m², and a period of time until the luminance reached 80% of the initial luminance was 3000 hours.

### Comparative Example 1

An organic EL device was fabricated in the same manner as in Example 1 except that the compound (A-5) was used instead of compound (A-1) when an emitting layer was formed.

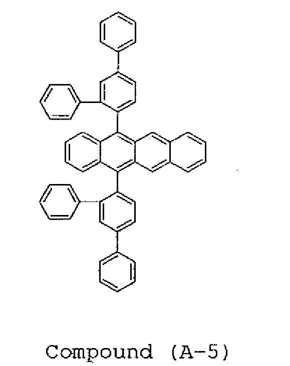

For the device thus obtained, a current test was performed. Red emission with a driving voltage of 4.1 V and luminance of 1135 cd/m² was obtained at a current density of 10 mA/cm². The chromaticity coordinates were (0.67, 0.33) and the efficiency was 11.35 cd/A. A direct current continuous test was performed at an initial luminance of 5000 cd/m², and a period of time until the luminance reached 80% of the initial luminance was 2100 hours.

### Industrial Applicability

The organic EL device of the invention can be used in the fields of various displays, back light, light source, indicators, signboards, interiors and the like, and it is particularly suitable for a display device of color displays.

## Claims

1. A naphthacene derivative represented by the following formula (1) or (2): 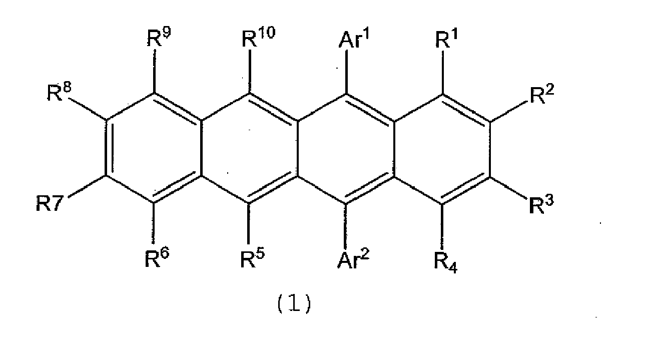 wherein Ar¹ and Ar² are not the same as each other, and are a substituted or unsubstituted aromatic group having 6 to 50 ring carbon atoms; and R¹ to R¹⁰ are each independently a hydrogen atom, substituted or unsubstituted aromatic group having 6 to 50 ring carbon atoms, or substituted or unsubstituted alkyl group having 1 to 50 carbon atoms; 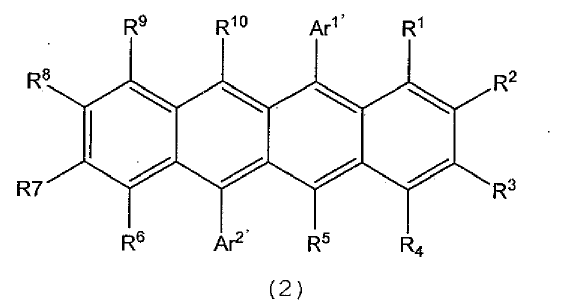 wherein Ar^{1'} and Ar^{2'} may be the same as each other, and are a substituted or unsubstituted aromatic group having 6 to 50 ring carbon atoms; and R¹ to R¹⁰ are each independently a hydrogen atom, substituted or unsubstituted aromatic group having 6 to 50 ring carbon atoms, or substituted or unsubstituted alkyl group having 1 to 50 carbon atoms.

2. The naphthacene derivative according to claim 1 which is selected from the group consisting of compounds represented by the following formulas (3) and (4): 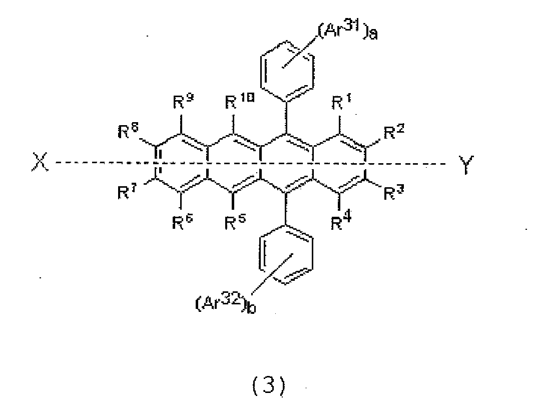 wherein Ar³¹ and Ar³² are each independently a substituted or unsubstituted aromatic group having 6 to 50 ring carbon atoms; R¹ to R¹⁰ are each independently a hydrogen atom, substituted or unsubstituted aromatic group having 6 to 50 ring carbon atoms, or substituted or unsubstituted alkyl group having 1 to 50 carbon atoms; and a and b are each an integar of 0 to 5, provided that groups do not symmetrically bond to 5 and 12 positions of the central naphthacene with respect to the X-Y axis; 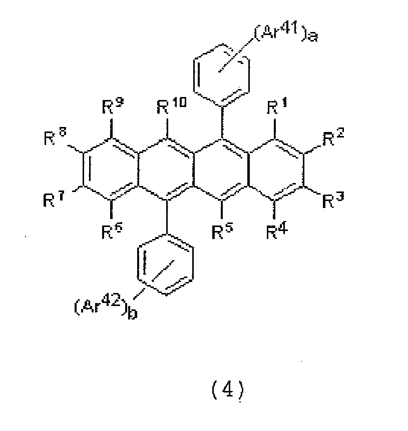 wherein Ar⁴¹ and Ar⁴² are each independently a substituted or unsubstituted aromatic group having 6 to 50 ring carbon atoms; R¹ to R¹⁰ are each independently a hydrogen atom, substituted or unsubstituted aromatic group having 6 to 50 ring carbon atoms, or substituted or unsubstituted alkyl group having 1 to 50 carbon atoms; and a and b are each an integer of 0 to 5.

3. A material for an organic electroluminescence device comprising the naphthacene derivative of claim 1 or 2.

4. A luminescent material for an organic electroluminescence device comprising the naphthacene derivative of claim 1 or 2.

5. An organic electroluminescence device comprising:
at least one organic layer interposed between a cathode and an anode, and
the organic layer containing the naphthacene derivative of claim 1 or 2.

6. The organic electroluminescence device according to claim 5 wherein the organic layer containing the naphthacene derivative of claim 1 or 2 is an emitting layer.

7. An organic electroluminescence device comprising:
an emitting layer and an electron-transporting layer between a cathode and an anode, and
the emitting layer containing a host material that is the naphthacene derivative of claim 1 or 2 and a dopant material that is an indenoperylene derivative.

8. The organic electroluminescence device according to claim 7 wherein the electron-transporting layer contains a compound represented by the following formula (5),
A-B (5)
wherein A is an aromatic hydrocarbon group with three or more carbocircles and B is a substituted or unsubstituted heterocyclic group.

9. The organic electroluminescence device according to claim 8 wherein the compound represented by the formula (5) is a compound containing in the molecule thereof at least one skeleton selected from anthracene, phenanthrene, naphthacene, pyrene, chrysene, benzoanthracene, pentacene, dibenzoanthracene, benzopyrene, fluorene, benzofluorene, fluoranthene, benzofluoranthene, naphthofluoranthene, dibenzofluorene, dibenzopyrene and dibenzofluoranthene.

10. The organic electroluminescence device according to claim 8 wherein the compound represented by the formula (5) is a nitrogen-containing heterocyclic compound.

11. The organic electroluminescence device according to claim 10 wherein the nitrogen-containing heterocyclic compound is a nitrogen-containing heterocyclic compound containing in the molecule thereof at least one skeleton selected from pyridine, pyrimidine, pyrazine, pyridazine, triazine, quinoline, quinoxaline, acridine, imidazopyridine, imidazopyrimidine and phenenthroline.

12. The organic electroluminescence device according to claim 10 wherein the nitrogen-containing heterocyclic compound is a benzoimidazole derivative represented by formula (6) or (7), 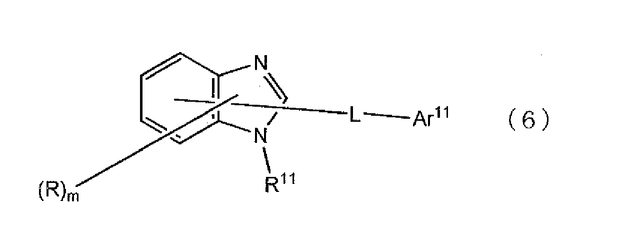 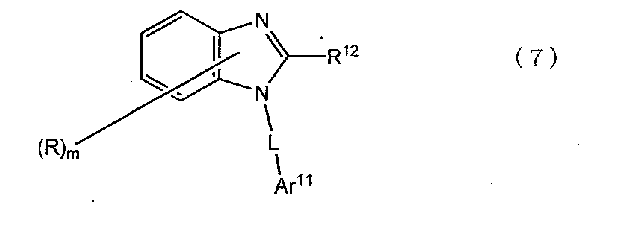 wherein R is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or substituted or unsubstituted alkoxy group having 1 to 20 carbon atoms;
m is an integer of 0 to 4;
R¹¹ is a substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or alkoxy group having 1 to 20 carbon atoms;
R¹² is a hydrogen atom, substituted or unsubstituted aryl group having 6 to 60 carbon atoms, substituted or unsubstituted pyridyl group, substituted or unsubstituted quinolyl group, substituted or unsubstituted alkyl group having 1 to 20 carbon atoms, or substiuted or unsubstituted alkoxy group having 1 to 20 carbon atoms;
L is a substituted or unsubstituted arylene group having 6 to 60 carbon atoms, substituted or unsubstituted pyridinylene group, substituted or unsubstituted quinolinylene group, or substituted or unsubstituted fluorenylene group; and
Ar¹¹ is a substituted or unsubstituted aryl group having 6 to 60 carbon atoms, substituted or unsubstituted pyridinyl group, or substituted or unsubstituted quinolinyl group.

13. The organic electroluminescence device according to any one of claims 7 to 12 wherein the indenoperylene derivative of the dopant material in the emitting layer is one or more compounds selected from the group consisting of indenoperylene derivatives represented by the following formulas (12) and (13): 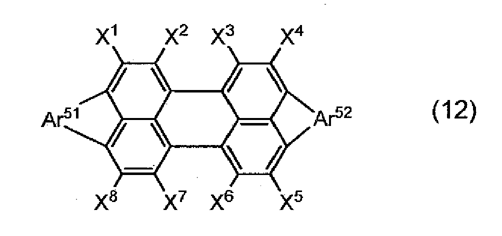 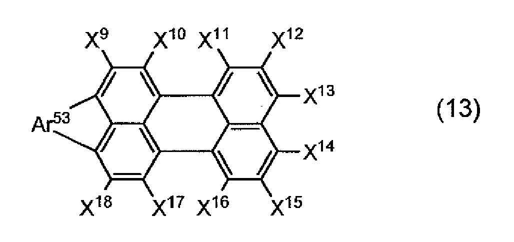 wherein Ar⁵¹, Ar⁵² and Ar⁵³ are each independently a substituted or unsubstituted aromatic hydrocarbon group having 6 to 50 ring carbon atoms, or substituted or unsubstituted aromatic heterocyclic group having 6 to 50 ring atoms; X¹ to X¹⁸ are each independently a hydrogen atom, halogen atom, substituted or unsubstituted alkyl group having 1 to 50 carbon atoms, substituted or unsubstituted alkoxy group having 1 to 50 carbon atoms, substituted or unsubstituted alkylthio group having 1 to 50 carbon atoms, substituted or unsubstituted alkenyl group having 2 to 50 carbon atoms, substituted or unsubstiuted alkenyloxy group having 1 to 50 carbon atoms, substituted or unsubstituted alkenylthio group having 1 to 50 carbon atoms, substiuted or unsubstituted aromatic hydrocarbon group having 6 to 50 ring carbon atoms, substiuted or unsubstiuted aromatic heterocyclic group having 6 to 50 ring atoms, substiuted or unsubstiuted aryloxy group having 6 to 50 ring carbon atoms, substituted or unsubstiuted arylthio group having 6 to 50 ring carbon atoms, substiuted or unsubstiuted aralkyl group having 7 to 50 ring carbon atoms, substiuted or unsubstituted arylalkyloxy group having 6 to 50 ring carbon atoms, substiuted or unsubstiuted arylalkylthio group having 6 to 50 ring carbon atoms, substiuted or unsubstiuted arylalkenyl group having 6 to 50 ring carbon atoms, substiuted or unsubstiuted alkenylaryl group having 6 to 50 ring carbon atoms, amino group, carbazolyl group, cyano group, hydroxy group, -COOR⁵¹, -COR⁵² or -OCOR⁵³ (R⁵¹, R⁵² and R⁵³ are each a hydrogen atom, substituted or unsubstituted alkyl group having 1 to 50 carbon atoms, substituted or unsubstituted alkenyl group having 2 to 50 carbon atoms, substituted or unsubstituted aralkyl group having 7 to 50 ring carbon atoms, substituted or unsubstituted aromatic hydrocarbon group having 6 to 50 ring carbon atoms, or substituted or unsubstituted aromatic heterocyclic group having 6 to 50 ring atoms); adjacent groups of X¹ to X¹⁸ may be bonded to each other to form a ring with a substituted carbon atom; and at least one of X¹ to X¹⁸ is not a hydrogen atom.

14. The organic electroluminescence device according to any one of claims 7 to 13 wherein the indenoperylene derivative of the dopant material in the emitting layer is a dibenzo tetraphenyl periflanthene derivative.

15. The organic electroluminescence device according to any one of claims 7 to 14 wherein the doping concentration of the dopant in the emitting layer is 0.1 to 10 wt%.

16. The organic electroluminescence device according to claim 15 wherein the doping concentration of the dopant in the emitting layer is 0.5 to 2 wt%.

17. The organic electroluminescence device according to any one of claims 7 to 16 whose emission color is orange to red.

18. An apparatus comprising the organic electroluminescet device of any one of claims 7 to 17.
